Europäisches Patentamt

European Patent Office

Office européen des brevets

(11) Publication number: **0 380 260**
**A2**

# EUROPEAN PATENT APPLICATION

(21) Application number: 90300609.6

(51) Int. Cl.5: **A61B 3/117, A61B 3/15**

(22) Date of filing: **22.01.90**

(30) Priority: **23.01.89 JP 12017/89**

(43) Date of publication of application:
**01.08.90 Bulletin 90/31**

(84) Designated Contracting States:
**CH DE FR GB IT LI**

(71) Applicant: **KOWA COMPANY LTD.**
**6-29, Nishiki 3-chome**
**Naka-ku Nagoya-shi Aichi-ken, 460(JP)**

(72) Inventor: **Akiyama, Koichi**
**1-17-11, Asahigaoka**
**Hino-shi, Tokyo 191(JP)**

(74) Representative: **Miller, Joseph et al**
**J. MILLER & CO. Lincoln House 296-302 High**
**Holborn**
**London WC1V 7JH(GB)**

(54) **Ophthalmic measurement apparatus.**

(57) An ophthalmic measurement apparatus in which a laser beam is projected at a selected spot (P) in a patient's eye (E) and light scattered therefrom is photoelectrically detected through a mask (21) by a photodetector (22) and processed for ophthalmic measurement. The apparatus is provided with an alignment index (41), which is disposed at a position conjugate with the mask and projected on the selected spot. The alignment index appears to be on the point of converged laser light and a pseudo-image (21a) of the mask can be observed at the point (P) in the eye where the laser light converges. This enables the system to be aligned with ease relative to the eye to be examined.

FIG. 2

## OPHTHALMIC MEASUREMENT APPARATUS

BACKGROUND OF THE INVENTION

Field of the Invention

This invention relates to an ophthalmic measurement apparatus, and more particularly to an ophthalmic measurement apparatus in which a laser beam is projected into the interior of a patient's eye and light scattered from the eye is detected for ophthalmic diagnosis measurement.

Description of the Prior Art

Measurement of protein concentration in the aqueous chamber of the anterior camera oculi is of considerable importance in determining whether the camera oculi is inflamed, that is, whether the blood-aqueous barrier function is normal or not. A number of methods are employed for carrying out this measurement. In one method that is frequently used, a slit lamp microscope is employed to grade the concentration by observation with the naked eye, while another method that has been reported uses photographic techniques to provide quantitative measurements. However, as yet there is no method that is easy to use clinically.

Data gained with the conventional method of naked-eye measurement lacks reliability, since judgments can vary depending on the person making the measurement. One solution has been to use a method in which laser light is projected into the eye and the light scattering from the eye is detected and analyzed quantitatively.

The extremely low intensity of the scattered laser light makes it susceptible to light other than light from the detection target, which manifests itself as noise. Taking the detection of the anterior chamber as an example, if the detection area is too close to the crystalline lens, light scattering from the crystalline lens is picked up as noise that influences the detection results.

Also, because the cornea has a strong lens effect, light other than light impinging from the normal line is refracted by the cornea surface. Hence, when the area on which the light impinges is changed the degree of refraction also changes, disturbing the relationship between the detection area (on which laser light converges) and the light receiving means (mask).

With the depth of the aqueous humor in the anterior chamber being around 3 mm, the laser has to be focused on a median portion measuring 1 mm to 2 mm and the light scattered from this measurement area has to be accurately captured. This requires accurate alignment of the apparatus with the patient's eye, particularly in the horizontal plane, and a method of achieving this alignment accurately.

SUMMARY OF THE INVENTION

An object of the present invention is therefore to provide an ophthalmic measurement apparatus which enables the apparatus to be readily aligned with the patient's eye.

In the present invention the above object is achieved by providing an illuminated alignment index for aligning an eye under examination with the apparatus, the alignment index being provided at a position that is conjugate with the position of a mask for limiting the field of vision that is provided in front of a photodetector for detecting scattered laser light from the eye under examination.

With this arrangement, since the position of the converged point of laser light and that of the alignment index are each conjugate with respect to the mask, the alignment index appears to be on the point of converged laser light to a person conducting an examination of the eye, and thus a pseudo-image of the mask, that is, an image of the illuminated alignment index, is observed at the point where the laser light converges. The fact that a pseudo-image of the mask can be observed at the point where the laser light beam converges means that it is possible directly to observe the actual point of origin of scattered light entering the photodetector. It also means that it is possible to align the system so that harmful rays of light from sources such as the cornea and the crystalline lens that have no relationship with the part being measured are directed outside the area of the mask pseudo-image. Thus, harmful light rays can be blocked by aligning the system appropriately, thereby increasing the effective component in the measured signal and improving measurement accuracy.

BRIEF DESCRIPTION OF THE DRAWINGS

The purposes and features of the present invention will become more apparent from a consideration of the following detailed description taken in conjunction with the accompanying drawings in which:

Figure 1 is a perspective view showing a structural arrangement of the ophthalmic measurement apparatus of the present invention;

Figure 2 is a side view of the internal configuration of the projection section of the apparatus shown in Figure 1;

Figure 3 shows details of the configuration of the light receiving section;

Figure 4 is an explanatory drawing of the observed image around the point of converged light in the observation section of the apparatus of Figure 1;

Figures 5a to 5c and 6a to 6c are explanatory drawings illustrating how alignment is determined in the apparatus of Figure 1;

Figure 7 shows the positional arrangement of the light projection section and the light receiving section during measurement;

Figure 8 is an explanatory drawing of the alignment index; and

Figure 9 and Figures 10a to 10c are explanatory drawings illustrating how alignment is determined in the apparatus of Figure 1.

## DETAILED DESCRIPTION OF THE PREFERRED EMBODIMENTS

The invention will now be described in detail on the basis of the preferred embodiments illustrated in the drawings.

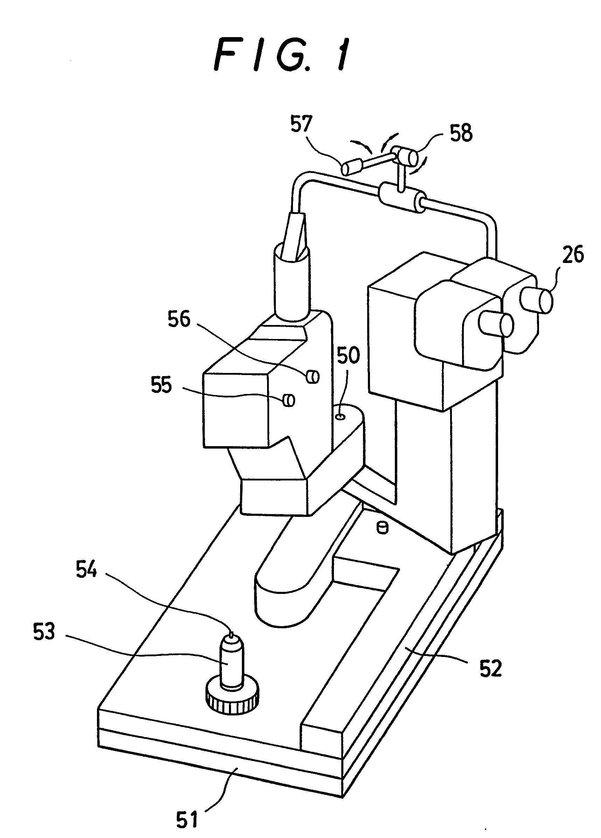

Figures 1 to 3 show the general arrangement of the embodiment of the ophthalmic measurement apparatus of this invention. In the drawings, reference numeral 1 denotes a laser light projection section housing a semiconductor laser or other such laser light source 3. The laser beam from the laser light source 3 is formed into an elliptical parallel beam by a collimating lens 4 and is then formed into a round parallel beam by a beam expander constituted by lenses 5 ad 6. After passing through a relay lens 7, an optical scanner 8, a beam-splitter 9, a lens 10 and a prism 11, the beam is converged on a predetermined point P in the anterior chamber of the eye under examination E.

The optical scanner 8 is connected to a drive circuit 30 controlled by a control section 31 constituted by a microprocessor or the like, to form an arrangement that allows the angle of the optical scanner 8 to be adjusted. The laser beam is deflected about a center formed by the point of convergence P by adjusting the angle of the optical scanner 8.

The laser light projection section 1 is provided with a halogen lamp or other such light source 12 for illuminating a slit 14. White light from the light source 12 illuminates a slit 14 via a lens 13. The light from the slit 14 thus illuminated passes via a shutter 15, the beam splitter 9, the lens 10 and the prism 11 to form a slit image in the vicinity of the point of convergence P in the anterior chamber of the eye.

By illuminating the area around the point of convergence P, the slit image allows the position of the point of convergence P to be readily confirmed when measuring the protein concentration in the anterior chamber. The slit image also serves to illuminate the cornea when the alignment index is being aligned with the cornea.

The width and length of the slit 14 can be adjusted by a slit width adjustment knob 56 and slit length adjustment knob 55 to enable the apparatus to be utilized also as a slit-lamp microscope.

The shutter 15 is controlled so that it is open during alignment and closed during protein concentration measurement. This comprises inserting the shutter 15 into, or retracting it from, the corresponding optical system by operating an input device such as a joystick 53 equipped with a push-button switch 54, which is mounted on a base 51.

A light receiving section 2 is provided for receiving scattered light from the vicinity of the point of convergence P and to allow the area to be observed. For this, scattered light from the point of convergence P in the anterior chamber of the eye under examination E passes through a lens 16, is reflected by a semi-transparent mirror 17 and passes, via a lens 18, interference filter 19, shutter 20 and mask 21 to a photomultiplier 22 which constitutes the photodetector. The interference filter 19 is a narrow-band interference filter with a peak wavelength that is the wavelength of the light of the laser light projection section 1 and blocks external light, to enable it to be used in a half darkroom. The field of view is defined by a mask 21 which prevents light outside the region of interest from impinging on the photomultiplier 22, for which the mask 21 is provided at a position that is conjugate with the point of convergence P in the light receiving section 2.

The output from the photomultiplier 22 is passed through an amplifier 23 and input to a counter 33 which is connected to a control section 31. The counter 33 measures the intensity of scattered light detected by the photomultiplier as a pulse count per unit time period. The output of the counter 33, i.e., the number of samplings or the total pulse count for each unit time period, is stored in a specific memory cell of a memory 32. The data thus stored in the memory 32 is arithmetically processed by the control section 31 to compute the protein concentration in the anterior chamber.

The shutter 20 is provided to protect the photomultiplier 22 and is open only during measurement. Like the shutter 15, it is inserted into, or retracted from, the corresponding optical system by operating an input device such as the joystick 53 equipped with a push-button switch 54.

Provided to the rear of the semi-transparent mirror 17 of the light receiving section is an observation section which allows a region in the eye centering on the point of convergence to be observed. Specifically, light that is split by passing through the semi-transparent mirror 17 can be observed by an examiner 29 via a lens 24, erect prisms 25 and 26, field stop 27 and eyepiece 28. This observation section allows the state of the projected laser beam and harmful light rays to be observed when measuring the protein concentration.

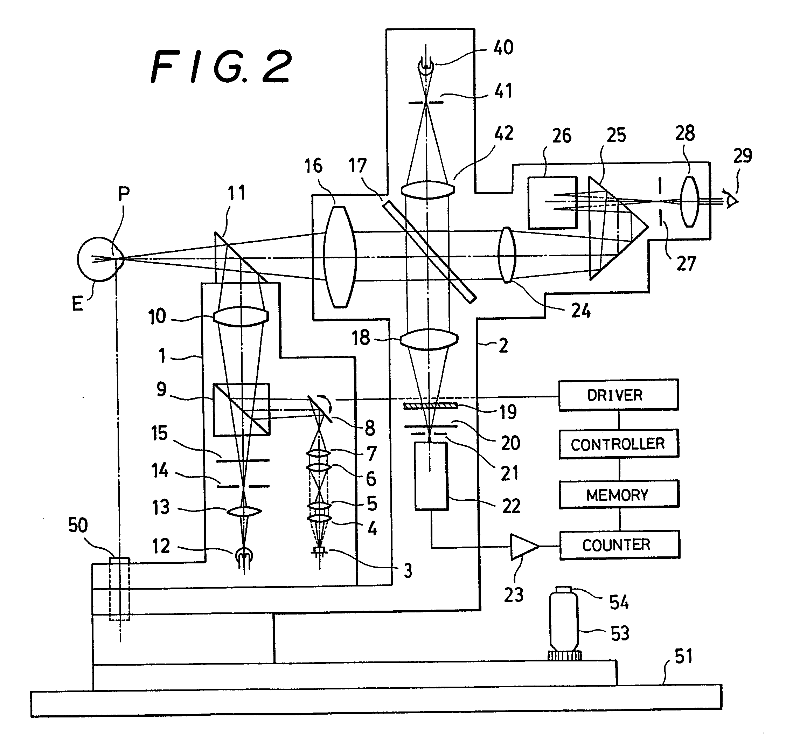

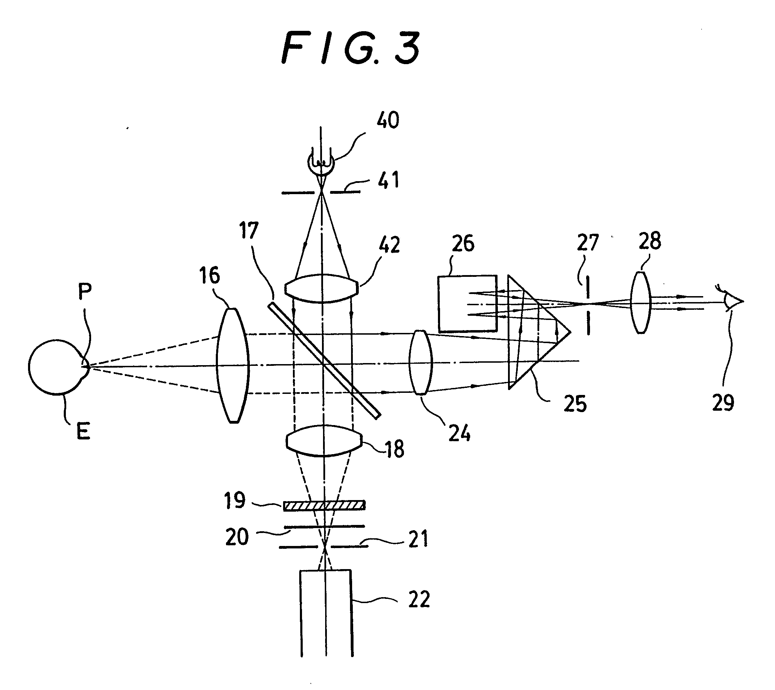

As shown in Figures 2 and 3, provided on the light receiving section 2 is an alignment index 41 which is illuminated by a light-emitting diode or other such light source 40. The alignment index 41 is disposed at a position that is conjugate with the mask 21 and the field stop 27. Thus, point of convergence P, mask 21 and field stop 27 are conjugate, as are alignment index 41, mask 21 and field stop 27.

An eye fixation light 57 constituted, in this embodiment, by a light-emitting diode is provided at a position that permits the examiner to fix the patient's eye. The eye fixation light 57 can be turned in the direction indicated by the arrow by means of a link mechanism 58 to enable it to be adjusted to the best position with respect to the patient undergoing the eye examination. The light selected for the eye fixation light 57 is of a different color than the laser light.

Provided on a base 51 is an input means such as the joystick 53 equipped with a push-button 54 which, as described above, is used for moving optical elements such as the shutters 15 and 20 into and out of the optical system concerned. The laser light projection section 1 and light receiving section 2 can each rotate independently in a horizontal plane about a shaft 50. When measuring protein concentration in the anterior chamber the laser light projection section 1 and the light receiving section 2 are fixed at an angle of 30 degrees and 90 degrees with respect to the normal at the top of the cornea, respectively. To use the apparatus as a slit-lamp microscope, the two sections are unlocked to allow them to rotate freely to view a cross-section of the eye.

A power supply housing 52 contains various components and circuitry including the control section 31, memory 32, counter 33 and power supply.

The overall alignment and measurement procedures will now be described. After positioning the patient's head, using a conventional chin rest (not illustrated), the slit light source 12 is switched on to project an image of the slit 14 onto the eye under examination E, and the laser beam from the laser light projection section 1 is converged to the point of convergence P in the eye. Next, the alignment light source 40 is switched on to illuminate the alignment index 41.

The alignment index 41 is disposed at a position that is conjugate with the mask 21 and the field stop 27, so the mask 21 and field stop 27 are conjugates of the point of convergence P, and the mask 21 and field stop 27 are conjugates of the alignment index 41. As a result, an image of the alignment index 41 illuminated by the light source 40 is formed at the field stop 27 and mask 21 which are conjugate points with respect to the alignment index 41. In addition, as the field stop 27 and the mask 21 are conjugates with respect to the point of convergence P, the alignment index 41 appears to be on the point of convergence P to the examiner 29.

When an alignment index 41 is used that has the same size and shape as the mask 21, the examiner can observe the mask 21 as if it were at the point of convergence P. Here, this observed image is termed the pseudo-image. Light originating in an area outside the pseudo-image will not be able to pass through mask 21, so by aligning the system as described below, harmful rays of light from sources such as the cornea and the crystalline lens can be directed outside the area of the mask pseudo-image to thereby suitably block harmful light rays.

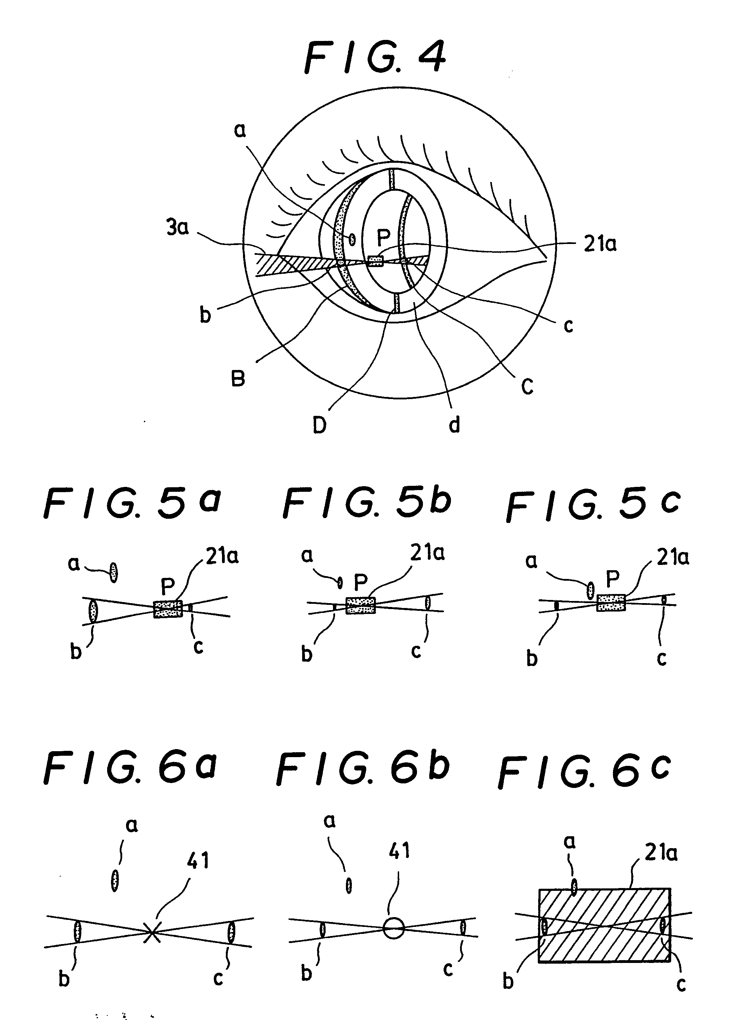

That is, an image of the alignment index 41 illuminated by the light source 40 is formed at the field stop 27 via lens 42, semi-transparent mirror 17 lens 24 and erect prisms 25 and 26, and the examiner 29 can observe through the eyepiece 28 the image appears to be at the point of convergence P. Figure 4 illustrates how a pseudo-image 21a of the mask is formed at the point of convergence P of a laser beam 3a. The laser beam 3a impinging on the cornea before reaching the point of convergence P causes laser light to scatter from the part of the cornea marked b. B denotes the front and rear surfaces of the cornea illuminated by the slit light. Slit and laser light is therefore actually scattered from two points, the front and rear surfaces of the cornea, but these are in close proximity, and in the drawing are therefore denoted as a single point (b).

As the laser beam 3a passes through the point of convergence P to impinge on the crystalline lens, the laser beam 3a produces scattered light from point c on the crystalline lens. C is scattered light from the crystalline lens illuminated by slit light, d is the iris; and D is the iris surface illuminated by slit light. At a, a corneal image is formed by scattered light from the exit face of the prism 11. This is because as the laser light passes through the prism 11 and converges in the eye, the laser light is scattered by the faces of the prism, producing secondary light sources that give rise to spurious images owing to the convex mirror effect of the cornea.

The above points a, b and c are the main sources of harmful rays (if the laser beam does not impinge directly on the iris, there will be no c). Alignment is performed to prevent harmful rays from points a, b and c from coming within the pseudo-image 21a. a, b and c behave like scattered light sources with low directionality and illuminate the vicinity, so in order to receive scattered light only from protein in the anterior chamber, the system should preferably be aligned to separate the harmful light sources a, b and c as much as possible.

Figure 4 shows an ideal state of alignment and Figure 5 shows an undesirable state. Figure 5a shows when the point of convergence P is too close to the crystalline lens, 5b when it is too close to the cornea and 5c when the laser beam is too high.

Thus, using the observation section, the alignment is performed to separate the harmful light ray sources a, b and c from the pseudo-image 21a by as much as possible. This permits scattered light from protein in the anterior chamber to be received and improves measurement accuracy. Making the light that illuminates the alignment index a different color from the laser light enables the mask pseudo-image to be readily distinguished from the harmful light ray sources a, b and c.

Using the same size lens for the lenses 12 and 18 to provide a conjugate magnification relationship, or using a alignment index that is the same size and shape as the mask increases an observer's impression that the mask 21 is at the point of convergence P. However, alignment is possible without such use of magnification or an alignment index of the same size and shape as the mask.

For example, as shown in Figure 6a, the alignment index could be in the form of crosshairs, or the mask could be round, as in Figure 6b. Also, if too high a magnification is used the pseudo-image 21a will cover the harmful light ray sources a, b and c as shown in Figure 6c, while too low a magnification will make it hard to perceive the pseudo-image, so the magnification should be set to a suitable level. When magnification is used, if harmful rays cross the mask pseudo-image even slightly, the mask itself is crossed, so preferably measurement should not be carried out in that state.

The harmful light ray sources a, b and c can be produced by reflection as well as by scattering. Therefore, there are also harmful rays reflected by the surface of the crystalline lens back to the cornea. The surface of the human crystalline lens does not give rise to much reflection. However, depending on the attachment methods artificial crystalline lenses can reflect light near the mask pseudo image. In such cases, therefore, reflections from artificial crystalline lenses have to be avoided in addition to harmful light ray sources a, b and c.

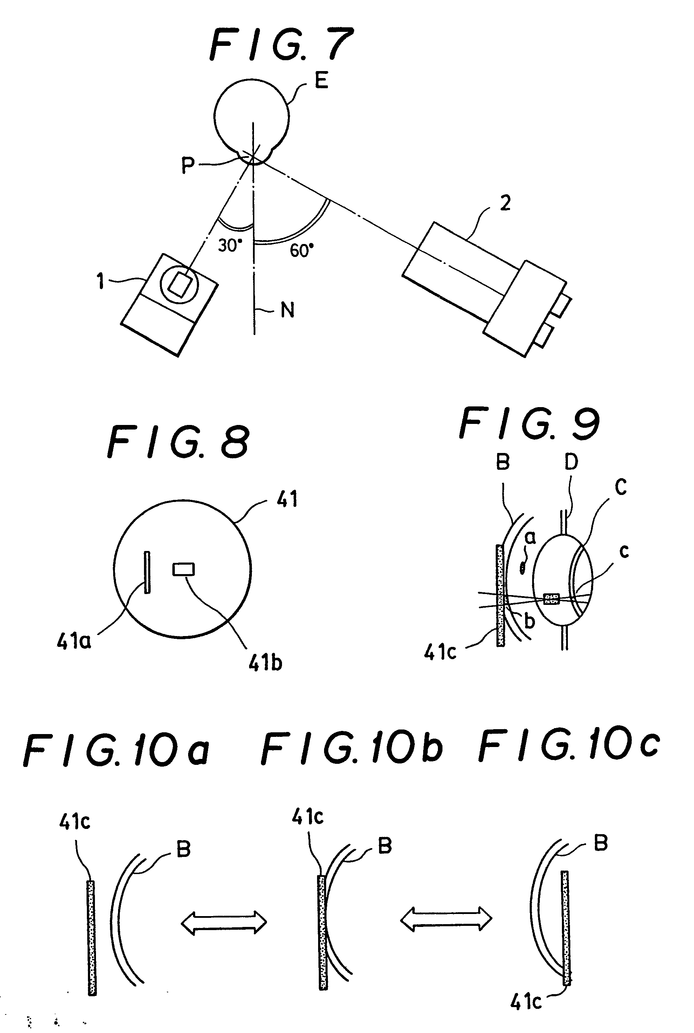

With respect to actual measurement, as shown in Figure 7 the projection section 1 is set at an angle of about 30 degrees and the light receiving section 2 at an angle of about 60 degrees to the normal N of the point of the cornea, so alignment along the axis of the light receiving section 2 results in a decrease in sensitivity. Because of this, as shown in Figure 8 the alignment index 41 is provided with a slit 41a at a position where the cornea will be. Numeral 41b denotes a mask for forming a pseudo image.

The image thus observed via the observation section is shown in Figure 9. Here, 41c is an image of the slit 41a. This image 41c is aligned with the slit image B of the cornea slit-illuminated by the projection section 1. As the slit light source 12 is a halogen lamp and the laser light beam is red, if the alignment index light source 40 is green, the harmful light ray sources a, b and c will be red and the slit image 41c will be green, while each of the slit illuminated portions B, C and D will be white, enabling them to be readily distinguished.

Figure 10 shows the relationship between the slit image 41c and the slit-illuminated image when the eye is moved toward the light receiving section. Figures 10a and 10C illustrate undesirable examples and Figure 10b a desirable example in which alignment can be carried out easily and accurately.

After the above alignment is achieved, switch 43 of a joystick 42 is pressed to close shutter 15 and open shutter 20 and scattered laser light (projected by the projection section 1) is measured via the light receiving section 2 to determine the protein concentration in the anterior chamber.

The projection section 1 projects the beam of laser light at the point of convergence P of the eye E under examination, and light reflecting from the region around the point of convergence P is received by the light receiving section 2. The output from the photomultiplier 22 is passed through the amplifier 23 and input to the counter 33 which is connected to the control section 31. The counter 33 measures the intensity of the scattered light detected by the photomultiplier 22 as a pulse count per unit time period. The output of the counter 33, i.e., the number of samplings or the total pulse count for each unit time period, is stored in a specific location of the memory 32. The data thus stored in the memory 32 is arithmetically processed by the control section 31 to compute the protein concentration in the anterior chamber. As this measurement procedure is conventional, further details thereof are omitted.

When using the apparatus as a slit-lamp microscope, a cross-section of the eye is viewed. During this procedure the laser is switched off, both because it is not required and to lengthen the working life of the laser. However, helium-neon lasers require time to stabilize after being reactivated, so when a helium-neon laser light source is used it is preferable to use a shutter or suchlike means to block the laser beam rather than deactivate it.

As has been described in the foregoing, in this invention an illuminated alignment index for aligning an eye under examination with the apparatus is provided at a position that is conjugate with the position of a mask for limiting the field of view that is positioned in front of a photodetector which detects scattered laser light from the eye under examination. Therefore, the alignment index appears to be on the point of converged light to the person conducting the examination, and thus a pseudo-image of the mask, that is, an image of the illuminated alignment index, is observed at the point of converged light.

This enables direct observation of the actual point of origin of scattered light entering the photodetector and also makes it possible to align the system so that harmful rays of light from sources such as the cornea and the crystalline lens that have no relationship with the part being measured, are directed outside the area of the mask pseudo-image. Thus, harmful light rays can be blocked by aligning the system appropriately, thereby increasing the effective component in the measured signal and achieving a higher level of measurement accuracy.

While the invention has been described with reference to a preferred embodiment, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention should not be limited to the particular embodiment disclosed as the best mode contemplated for carrying out the invention, but that the invention will include all embodiments falling within the scope of the appended claims.

## Claims

1. An ophthalmic measurement apparatus in which a laser beam (3a) from a laser source is projected at a selected spot (P) in a patient's eye (E) and light scattered therefrom is photoelectrically detected through a mask (21) by a photodetector (22) and processed by processing means ( 31 to 33) for ophthalmic measurement, characterized in that an alignment index (41) is disposed at a position that is conjugate with that of the mask (21), and that alignment index is projected on the selected spot in the eye for aligning the eye under examination with the apparatus.

2. An ophthalmic measurement apparatus as set forth in claim 1, wherein the alignment index (41) is substantially the same in size and shape as the mask (21).

3. An ophthalmic measurement apparatus as set forth in claim 1 or claim 2, wherein the alignment index (41) is illuminated by a light source (40) which provides light that is a different color from the laser light.

4. An ophthalmic measurement apparatus as set forth in any of claims 1 to 3, wherein the alignment index and the mask are in conjugate relationship in equal magnification size.

# F I G. 1

FIG.2

FIG. 3

# FIG. 4

# FIG. 5 a

# FIG. 5 b

# FIG. 5 c

# FIG. 6 a

# FIG. 6 b

# FIG. 6 c

*F I G. 7*

*F I G. 8*

*F I G. 9*

*F I G. 10 a*   *F I G. 10 b*   *F I G. 10 c*